# EUROPEAN PATENT SPECIFICATION

(11) **EP 0 327 386 B1**
(45) Date of publication and mention of the grant of the patent: **01.06.1994**
(21) Application number: 89301074.4
(22) Date of filing: 03.02.1989
(51) Int. Cl.: C12M 1/34, G01N 17/00

(54) **Apparatus for microbiological monitoring**
Mikrobiologische Kontrollvorrichtung
Appareil de contrôle microbiologique

(30) Priority: 04.02.1988 GB 8802473
(43) Date of publication of application: 09.08.1989
(73) Proprietor: HOUSEMAN LIMITED, Burnham, Slough SL1 7LS (GB)
(72) Inventor: Schapira, Simon Francis David, London SW12 (GB); Eycott, Robin William, Bledlow Ridge Bucks HP14 4JN (GB); Richardson, William Patrick, Burnham, Bucks (GB); Robinson, Jim Scott, Colnbrook Bucks SL3 0PY (GB)
(74) Representative: Jones, Helen Marjorie Meredith

(56) References cited:
- GB-A- 1 546 041
- US-A- 3 174 332
- CANADIAN JOURNAL OF MICROBIOLOGY, vol. 27, 1981, Natl. Research Council of Canada, CA; W.F. McCOY et al., pp. 910-917#

## Description

The present invention relates to apparatus for monitoring the buildup of films of microorganisms on surfaces in aquatic systems, such as in heat exchangers, waste water transport and secondary oil recovery, particularly in recirculating water systems such as in cooling systems. In particular the invention relates to apparatus which is easier to handle than known apparatus of this type.

In all industrial, domestic and medical aquatic systems there are many factors which can create problems by reducing the efficiency of the process concerned or by creating toxicity problems. Water used in most of these systems will usually contain some form of living microorganisms which under the conditions in the systems concerned can multiply and cause a number of problems. Initially the microbes are introduced suspended in the water. These so-called planktonic microorganisms are relatively easy to detect and monitor and also to kill using conventional biocides. Although they can cause problems, for instance if the microorganisms are pathogenic, in general they do not significantly effect the overall efficiency of the system.

The planktonic microorganisms can however become adherent to internal surfaces in the system when they are known as "sessile". Sessile bacteria can proliferate and some bacteria generate so called slime composed mainly of polysaccharide and form a film upon the surface. These films have recently been recognised as contributing to inefficiencies in the systems for various reasons. For instance the films will reduce efficiency of conductive heat transfer through the surfaces and, since they are highly elastic, increase fluid frictional resistance at the surface dramatically. Furthermore some types of bacteria produce compounds which may be environmentally hazardous or may lead to corrosion of metal in the surface to which the film is attached. Sulphate reducing bacteria or SRB's in particular can give rise to serious corrosion in metal surfaces.

Bacteria in biofilms are in general found to be difficult to get rid of, partly because chemical biocides must penetrate the slime before they reach the target microorganisms deep within the films. Because of the problems that biofilms can create it is important to be able to detect the appearance of the biofilms in order to know when a dose of a suitable biocide is necessary to prevent development of films and to get rid of existing films, as well as to be able to detect the removal of such films after treatment with such a biocide. Since films which are so thin as to be invisible to the naked eye can cause severe problems it is desirable to be able to detect films by other means. The absence of significant numbers of planktonic bacteria does not necessarily indicate the absence of sessile bacteria.

One type of device for monitoring biofilm buildup is described in the Canadian Journal of Microbiology (1981), volume 27, pages 910 to 917, in which McCoy et al describe the use of a so-called Robbins device which comprises a tube through which water in a recycling circuit can flow. The tube has a plurality of ports in its walls, each being provided with a stud having a biofoulable surface and being capable of being retained in the port in fixed relationship with respect to the tube so that the biofoulable surface forms part of the internal surface of the tube. The studs may be removed from the ports after a desired time interval and the test surfaces by microscopy of the surfaces analysed for the growth of microorganisms or by removal of the microorganisms from the surfaces and subsequent estimation of the degree of growth. The number of microorganisms can be estimated for instance by physical or chemical means, e.g. by detection of bacterial ATP or by further culturing the microorganisms and analysing the products.

One problem with conventional Robbins devices is that it is difficult to remove the studs from their retaining means for analysis of the biofilm and, having removed the stud, it is difficult to handle such a small component aseptically to avoid cross-contamination.

Ruseska et al in Oil and Gas Journal, March 8th 1982 describe the removal of biofilm bacteria from the test surfaces of a Robbins device by scraping with a sterile scalpel. It is difficult to remove all traces of the film from the surface using a scalpel. In "Developments in Industrial Biology" (1982) chapter 53, McCoy and Costerton describe the removal of biofilm from studs by dropping the entire stud into a test tube containing water and metallic tumbling abrasive. The tube is vortexed for two minutes to abrade the film from the surface. A problem with the latter form of removal of biofilm is that film from other areas of the stud is also removed into the same water. This can lead to erroneous results since bacteria growing on such surfaces do not necessarily simulate growth on the internal surfaces of the rest of the recirculating water system, the conditions of flow and other apsects of their environment possibly being significantly different. It is practically impossible to prevent growth occurring on such surfaces but it would be desirable to be able to sterilise such surfaces without affecting the film on the test surface before its analysis.

Accordingly all the present forms of apparatus are difficult to handle or give results that are inevitably inaccurate, or both.

In US 3174332 an apparatus described as a Test Coupon Positioner is described which tests the effects of coal slurries pumped under pressure on metallic "coupons". The device allows coupons to be positioned in the bulk fluid flow within a pipeline and to be removed after predetermined periods for investigation of any corrosion or erosion of the coupon. The coupons may be removed by use of a removal member which has mounting means for connection with the coupon positioner.

According to the present invention there is provided a new kit for monitoring biofouling on the surfaces in aquatic systems comprising sampler apparatus comprising a duct which forms a channel for a flow of water and has at least one port (2) in its wall, a stud (17) having a biofoulable test surface (19) and retaining means (13) for retaining the stud in the port in fixed relationship with respect to the tube so that the test surface is flush with the wall and is exposed to water in the channel and the kit is characterised in that it also comprises a stud removal member (21) and the stud and stud removal member have mutual mounting means suitable to rigidly mount the stud on the stud removal member for removal from the apparatus.

The duct is usually a tube although it could be another type of duct through which water can flow and in the wall of which the retaining means can retain the stud.

The sampler apparatus is of particular use for monitoring the growth of sessile microorganisms on pipe and other duct walls in aquatic systems and so the stud is capable of being retained so that its test surface is flush with the duct wall. In such a position the test surface lies in the laminar flow or boundary layer region in the pipe.

Generally the sampler apparatus comprises a plurality of studs with respective retaining means. Some times the apparatus may comprise a single port in the duct and retaining means for retaining a plurality of studs within that port, for instance of the type sold by Caproco Corrosion Prevention Limited for on-line monitoring of circulating water systems. Preferably however the apparatus comprises a duct having a plurality of ports each having a respective stud held in position by respective retaining means. The duct may be connected in series with the system being monitored or, preferably, it is connected in parallel so that a portion only of the water from the system circulates through it. In such a system in which the duct forms a sidestream the duct may be closed off from the flow by isolating valves at each end of the duct whilst the main circulation through the system continues. The studs may then be examined optionally after disconnection of the entire sampler apparatus from the system.

Preferably the duct is capable of being connected into the circuit so that the conditions of water flow and temperature through the duct are independently adjustable The conditions can then be optimised for sessile bacterial growth so that biofouling happens earlier in the tube than in the rest of the system and so that the device can be used as a predictor of growth in the rest of the system.

Preferably the test surface of the stud is substantially flat in order to facilitate the analysis of growth. As in conventional Robbins devices, the stud can have a head with a flat test surface and a stem extending from the opposite side of the head. Preferably the back of the head is conical in shape which aids subsequent sterilisation of the stud and prevention of water migration during use.

In the sampler apparatus the stud is preferably retained in a sleeve as part of the retaining means. The sleeve can be shaped at one end for receiving the stud and minimising water access to surfaces of the stud other than the test surface for instance so that the test surface sits flush with the end of the sleeve. The sleeve may include a seal within its bore for maximising the seal with the stud.

Preferably the mounting means on the stud are such as to allow connection of the stud removal means from the side of the retaining means not facing the flow of water. For instance, a sleeve generally has a bore extending throughout its length through which the stud removal means may be inserted. The sleeve may be held in position in the port of the tube in the apparatus by any suitable cooperating fixing means, for instance so that it is removable from the port whilst still carrying the stud.

The stud removal means is preferably a rod and, if it must pass through a sleeve of the retaining means, as a diameter of the same or smaller than that of the stud stem.

In a further aspect of the invention there is provided handling apparatus comprising a stud, having a head with a test surface and a stem leading from the head, and a bored sleeve for receiving the stud with the test surface exposed and with the stem in a sliding fit in the bore, characterised in that the handling apparatus comprises also a removable rigid rod-shaped stud removal member insertable in and slidable through the sleeve bore and in that one end of the member and the stud stem have mutually cooperable mounting means which are capable of being brought into cooperation with each other whilst the stud is received in the sleeve to mount the stud on the member for handling before and after removal of the sleeve.

In the apparatus the stud may be held in the sleeve simply by friction between the contacting surfaces. Additionally or alternatively the stud may be retained within the sleeve by a pin or pins clips or other like means.

The mounting means via which the stud can be connected to the removal member may include screw threads provided on the stud and the removal member, a bayonet fixing, a push fit or other known alternative connecting means.

The components of the sampler apparatus are made of any suitable materials capable of withstanding corrosion in the system. Usually the stud is of metal, for instance aluminium or steel usually stainless steel. The tube may be made of metal, for instance copper or steel, or, convenently, of moulded plastics to avoid corrosion and scaling, for instance of moulded acrylonitrile butadiene styrene copolymer (ABS). The sleeve is made of for instance polymers and copolymers of ethylene and propylene, polytetra fluoroethylene or nylon, preferably nylon or polypropylene. The stud removal member can be made of metal or plastics materials, usually metal.

In the preferred embodiment of the invention, to remove the stud from its sleeve, one end of a rod shaped removal member is pushed through the open end of the sleeve and is fixed onto the stud via the mounting means. Then the sleeve is pushed along the length of the member to reveal the stud. The sleeve may be removed entirely or may be retained on the end of the rod distant from the stud as a handle. If the sleeve is removed a separate handle may be provided on the distal end of the stud removal member. The stud can then be treated by whatever means are necessary without the stud itself having to be handled. For instance the stud may have all of its surfaces other than the test surface swabbed using a sterilising composition in order to remove all other traces of bacteria. In the preferred embodiment, when the stud removal member is a rod that is attachable to the stem of a stud, the rod has approximately the same diameter as the stem to provide a continuous surface to aid swabbing. Then the biofilm on the test surface can be further analysed using the stud removal member to manipulate the stud as required. After analysis the stud can be discarded or can be sterilised and reused.

A preferred embodiment of the invention is illustrated in the drawings in which:
Figure 1 is a cross-section through a device in position in the sampler apparatus for biofilm formation (in the absence of the removal member);
Figure 2 is a cross-section through an embodiment of the handling apparatus comprising a stud still positioned in a sleeve that has been removed from the port and having a stud removal rod attached to the stud; and
Figure 3 is a cross-section through a stud mounted on a stud removal member having a handle at its distal end:
Figure 4 is an enlargement of part of figure 2 showing extra detail.

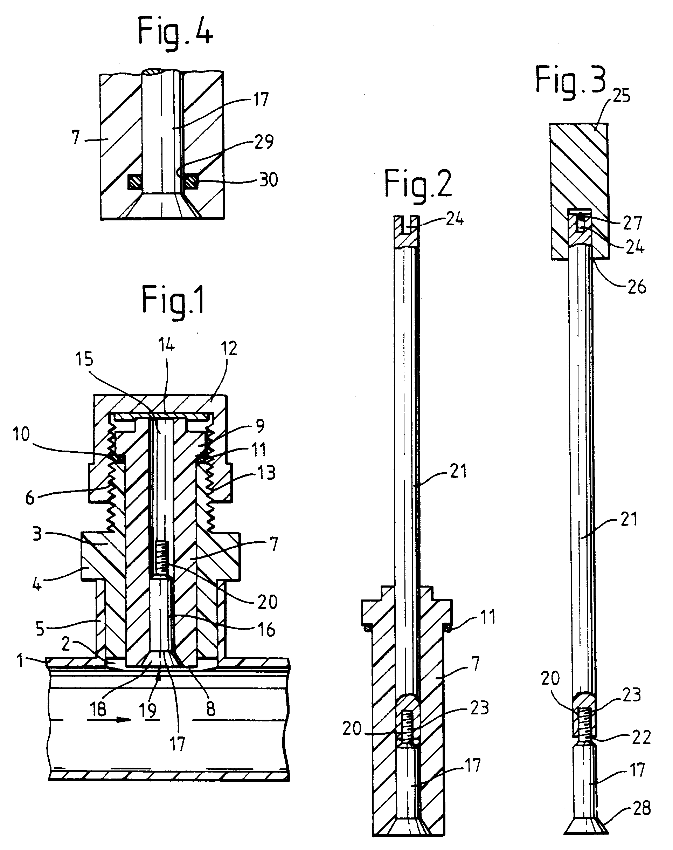

Referring to Figure 1, a tube 1 which provides a channel for flow of water as indicated by the arrow, has a port 2 in its wall. The tube is formed of moulded acrylonitrile butadiene styrene copolymer. The port receives a collar 3 of plastics or metal having a flange 4 which bears against the lip 5 around the port. Beyond the flange the collar has screw threads 6 on its external surface.

Within the collar is received a sleeve 7 formed of polypropylene or nylon whose end 8 directed towards the tube is approximately level with the internal surface of the wall of the tube. The sleeve has an external flange 9 at its other end which bears against the end surface 10 of the collar via an O-ring 11 which provides a water-tight seal between the components. The sleeve is held within the collar by means of a screw cap 12 e.g. of metal which fits over the sleeve and has screw threads 13 which co-operate with the external screw threads 6 of the collar. The cap has a gasket 14 which provides a water tight seal between the cap and the open end of the sleeve even when the cap is only hand tightened. Where the monitor is exposed to possible interference, it can be provided with a tampter-proof device to prevent removal of the caps, for instance comprising lockable means.

The sleeve has a central bore 15 of approximately the same internal diameter as the external diameter of the stem 16 of a stud 17 formed of aluminium. The stud has a head 18 with sloping shoulders and a test surface 19 which, when in position in the sampler apparatus, forms part of the boundary of the flow channel through the tube. The bore 15 of the sleeve is shaped at the inner end of the sleeve to receive the shaped stud. As shown in figure 4, within the bore there may be an annular depression 29 for receiving an O-ring 30 to form an improved seal between the stud stem and the sleeve to help secure the stud in position and prevent leakage of water into the space behind the stud.

The end of the stud distal from the head has a reduced diameter and is provided with screw threads on its external surface. These form part of the mounting means for attachment to the removal member.

Before connection into a circuit all the components of the sampler apparatus are chemically and physically sterilised. In use water from the circulating system is directed through the tube at a temperature and rate such as to optimise growth conditions so that the device can be used for sessile microorganisms. Microorganisms will attach to surface 19 of the stud, inter alia, and may start to form a film. Usually apparatus comprises a tube having a plurality of ports with associated collars, sleeves, caps and studs. Development of biofilm may be monitored by removal of one or more studs after a predefined interval after commencement of the monitoring process.

Removal of the stud from the embodiment shown in the drawings can then be carried out with the help of the stud removal member 21 as illustrated in Figures 2 and 3 of the drawings. The rod like member has at one end an aperture 22 whose internal surface is provided with screw threads 23 which can co-operate with the screw threads 20 on the stud. The other end of the member is provided a notch 24. A handle for the member 25 is provided with an opening 26 for receipt of the notched end of the member 21. The opening in the handle has a pin 27 which is capable of co-operating with the notch 24 on the member to retain the handle on the member.

To remove the stud from the apparatus, first of all the flow of liquid through the channel is stopped. Then the screw cap 12 is removed from the collar. This allows removal from the collar of the sleeve 7 still with the stud 17 retained within its bore. In this and subsequent operations care is taken not to allow the test surface 19 to come into contact with other items. The removal member 21 with or without its handle is inserted through the open end of the sleeve and is connected to the stud by means of the mutually co-operating screw threads. When these are completely coupled the handle, if on the rod, is removed to provide the apparatus as illustrated in Figure 2. As can be seen from the drawing the sleeve may be slid along the length of the member 21 and removed altogether. The handle may then be reapplied to the end of the rod.

The stud is now mounted on the removal member, its rigid mounting enabling it to be handled easily, as illustrated in Figure 3.

To analyse the deposit on the test surface, it is first preferable to sterilise the other stud surfaces, which are likely to develop some bacterial growth. This may be done by wiping the external surface of the rod and of the stud stem as well as the batch surfaces of the head 28 using alcohol or other sterilising composition. The shoulders on the back of the head are sloping to make satisfactory swabbing of the surfaces easier.

The biofilm on the test surface may be removed from the stud by conventional means, for instance by scraping the film with a scalpel by ultrasonic irradiation or by the method described by McCoy and Costerton (op.cit.). One advantage of the present invention is that it allows an easier and more effective and consistent way of removing the film. The stud still mounted on the removal member is plunged into a sterile vessel containing sand and water and is moved so as to grind the film from the test surface into the sand. When all the film has been removed the stud may be lifted from the water still attached to the rod. This method of removal of biofilm avoids tumbling of the entire stud in an abrasive and water containing tube. All or a sample of the water may then be analysed by conventional microbiological testing procedures. For instance the number of viable microorganisms in the sample and thus in the film may be estimated and/or identified by culturing methods. The number of bacteria may also be estimated by microscopic methods or by the ATP assay method described by McCoy and Costerton (op cit). Alternatively the film on the test surface of the stud may be analaysed by microscopic techniques, for instance by electron microscopy, fluorescence microscopy or light microscopy.

The kit according to the invention enables the apparatus to be used by operators who are relatively unskilled in microbiological techniques for instance by the engineers or chemists normally responsible for running and monitoring the water system.

## Claims

1. A kit for monitoring biofouling on surfaces in aquatic systems which comprises sampler apparatus comprising a duct which forms a channel for a flow of water and has at least one port (2) of its wall, a stud (17) having a biofoulable test surface (19) and retaining means for retaining the stud in the port in fixed relationship with respect to the duct so that the test surface is substantially flush with the duct wall and is exposed to water in the channel, characterised in that the kit also comprises a stud removal member (21) and the stud and stud removal member have mounting means suitable to rigidly mount the stud on the stud removal member for removal from the apparatus.

2. A kit according to claim 1 which comprises several studs with respective retaining means.

3. A kit according to any preceding claim in which the duct has several ports (2) in its wall each having an associated stud (17) and respective retaining means (13).

4. A kit according to any preceding claim in which the or each stud (17) comprises a head (18) one side of which forms the test surface (19) and from the opposite side of which extends a stem (16), the stud preferably having a sloping shoulder between the stem and the head.

5. A kit according to any preceding claim in which the retaining means comprises a sleeve (7) which has a stud retaining bore (15) and which is itself suitable for being retained in the respective port.

6. A kit according to any preceding claim in which the stud removal member (21) comprises a rod, and the mounting means preferably comprises a screw threaded connection (23), a bayonet fixing or a push fit connection between the stud and the stud removal member.

7. A kit according to claim 4 and claim 6 in which the stem and the rod have substantially equal diameters.

8. A kit according to any preceding claim which comprises also a handle (25) suitable for being detachably connected to the stud removal member, preferably at the end of the stud removal member distant from the stud mounting means, for instance for holding and manipulating the stud removal member (21) during the mounting of the stud (17).

9. A kit according to any preceding claim which is capable of being connected in parallel with the system being monitored and, preferably has an isolating valve at each end.

10. A method for monitoring biofilm formation on surfaces in aquatic systems which comprises incorporating in the aquatic system a sampler apparatus comprising a duct which forms a channel for a flow of water and has at least one port (2) in its wall, a stud (17) having a biofoulable test surface (19) and retaining means (13) for retaining the stud in the port into fixed relationship with respect to the duct so that the test surface is substantially flush with the duct wall and is exposed to water in the channel, and
retaining the stud in the sampler apparatus for an interval for testing, characterised in that the stud is subsequently removed from the port using a stud removal member (21), the stud and stud removal member having mounting means suitable to rigidly mount the stud on the stud removal member for removal from the apparatus, and
collecting and analysing the bacteria from the test surface of the stud.

## Patentansprüche

1. Anordnung zur Überwachung des Biobewuchses an Oberflächen im Wasser befindlicher Systeme, welche umfaßt eine Probenahme-Apparatur umfassend eine Rohrleitung, die einen Kanal für einen Wasserstrom bildet und in ihrer Wand mindestens eine Öffnung (2) aufweist, einen Bolzen (17), der eine biobewuchsfähige Prüfoberfläche (19) besitzt, und Halterungen, um den Bolzen fest verbunden mit der Rohrleitung in der Öffnung zu halten, so daß die Prüfoberfläche im wesentlichen bündig mit der Rohrleitungswand ist und dem Wasser in dem Kanal ausgesetzt ist, dadurch gekennzeichnet, daß die Anordnung ferner ein Bolzenentnahmeelement (21) umfaßt und der Bolzen und das Bolzenentnahmeelement Befestigungseinrichtungen aufweisen, die dazu geeignet sind, den Bolzen zur Entnahme aus der Apparatur fest an dem Bolzenentnahmeelement zu befestigen.

2. Anordnung nach Anspruch 1, welche mehrere Bolzen mit entsprechenden Halterungen umfaßt.

3. Anordnung nach irgendeinem vorangehenden Anspruch, worin die Rohrleitung in ihrer Wand mehrere Öffnungen (2) aufweist, wobei jede einen zugehörigen Bolzen (17) und entsprechende Halterungen (13) besitzt.

4. Anordnung nach irgendeinem vorangehenden Anspruch, worin der bzw. jeder Bolzen (17) einen Kopf (18) umfaßt, dessen eine Seite die Prüfoberfläche (19) bildet und aus dessen gegenüberliegender Seite ein Schaft (16) herausragt, wobei der Bolzen zwischen dem Schaft und dem Kopf vorzugsweise eine schräge Schulter aufweist.

5. Anordnung nach irgendeinem vorangehenden Anspruch, worin die Halterung eine Buchse (7) umfaßt, die eine Bolzenhaltebohrung (15) aufweist und die selbst in der entsprechenden Öffnung gehalten werden kann.

6. Anordnung nach irgendeinem vorangehenden Anspruch, worin das Bolzenentnahmeelement (21) eine Stange umfaßt und die Befestigungseinrichtung vorzugsweise eine Schraubengewindeverbindung (23), einen Bajonettverschluß oder eine Schiebepassungsverbindung zwischen dem Bolzen und dem Bolzenentnahmeelement umfaßt.

7. Anordnung nach Anspruch 4 und Anspruch 6, worin der Schaft und die Stange im wesentlichen gleiche Durchmesser haben.

8. Anordnung nach irgendeinem vorangehenden Anspruch, welche ferner einen Griff (25) umfaßt, der lösbar mit dem Bolzenentnahmeelement, vorzugsweise an dem der Bolzenbefestigungseinrichtung entfernten Ende des Bolzenentnahmeelements, verbunden werden kann, um das Bolzenentnahmeelement (21) z.B. während der Befestigung des Bolzens (17) zu halten und handzuhaben.

9. Anordnung nach irgendeinem vorangehenden Anspruch, welche parallel mit dem zu überwachenden System verbunden werden kann und vorzugsweise an jedem Ende ein Absperrventil aufweist.

10. Verfahren zur Überwachung der Biobewuchsbildung an Oberflächen im Wasser befindlicher Systeme, welches umfaßt
den Einbau einer Probenahme-Apparatur umfassend eine Rohrleitung, die einen Kanal für einen Wasserstrom bildet und in ihrer Wand mindestens eine Öffnung (2) aufweist, einen Bolzen (17), der eine biobewuchsfähige Prüfoberfläche (19) besitzt, und Halterungen (13), um den Bolzen fest verbunden mit der Rohrleitung in der Öffnung zu halten, so daß die Prüfoberfläche im wesentlichen bündig mit der Rohrleitungswand ist und dem Wasser in dem Kanal ausgesetzt ist, in das im Wasser befindliche System und
Halten des Bolzens in der Probenahme-Apparatur über einen Zeitraum zur Überprüfung, dadurch gekennzeichnet, daß der Bolzen anschließend unter Verwendung eines Bolzenentnahmeelements (21) aus der Öffnung entnommen wird, wobei der Bolzen und das Bolzenentnahmeelement Befestigungseinrichtungen aufweisen, die dazu geeignet sind, den Bolzen zur Entnahme aus der Apparatur fest an dem Bolzenentnahmeelement zu befestigen, und
Sammeln und Analysieren der Bakterien auf der Prüfoberfläche des Bolzens.

## Revendications

1. Coffret pour surveiller l'apparition de salissures biologiques sur des surfaces dans des systèmes aquatiques, comprenant un appareil d'échantillonnage qui comprend un conduit qui forme un canal pour un écoulement d'eau et qui possède au moins un orifice (2) dans sa paroi, un plot (17) ayant une surface de test bio-salissable (19), et des moyens de retenue (13) destinés à retenir le plot dans l'orifice, dans une relation fixe par rapport au conduit, de sorte que la surface de test est en affleurement avec la paroi du conduit et qu'elle est exposée à l'eau dans le canal, caractérisé en ce que le coffret comprend également un élément d'enlèvement (21) pour le plot, le plot et l'élément d'enlèvement ayant des organes de montage convenables pour monter rigidement le plot sur l'élément d'enlèvement dans le but de son l'enlèvement hors de l'appareil.

2. Coffret selon la revendication 1, comprenant plusieurs plots avec des moyens de retenue respectifs.

3. Coffret selon l'une quelconque des revendications précédentes, dans lequel le conduit comporte plusieurs orifices (2) dans sa paroi, ayant chacun un plot (17) associé et des moyens de retenue respectifs (13).

4. Coffret selon l'une quelconque des revendications précédentes, dans lequel ledit plot ou chaque plot (17) comprend une tête (18) dont un côté forme la surface de test (19) et avec une tige (16) qui s'étend depuis le côté opposé de la tête, le plot ayant de préférence un épaulement incliné entre la tige et la tête.

5. Coffret selon l'une quelconque des revendications précédentes, dans lequel les moyens de retenue comprennent un fourreau (7) qui présente un trou de retenue (15) pour le plot et qui est lui-même propre à être retenu dans l'orifice respectif.

6. Coffret selon l'une quelconque des revendications précédentes, dans lequel l'élément d'enlèvement (21) pour le plot comprend une barre, et les organes de montage comprennent de préférence un raccordement à vis (23), une fixation à baïonnette ou un raccordement par poussée entre le plot et l'élément d'enlèvement du plot.

7. Coffret selon la revendication 4 et la revendication 6, dans lequel la tige et la barre ont sensiblement des diamètres égaux.

8. Coffret selon l'une quelconque des revendications précédentes, qui comprend également une poignée (25) propre à être raccordée de manière détachable à l'élément d'enlèvement du plot, de préférence à l'extrémité de l'élément d'enlèvement du plot distante des organes de montage pour le plot, par exemple afin de maintenir et de manipuler l'élément d'enlèvement du plot (21) pendant le montage du plot (17).

9. Coffret selon l'une quelconque des revendications précédentes, lequel est capable d'être raccordé en parallèle avec le système en cours de surveillance et qui comporte de préférence une valve d'isolement à chaque extrémité.

10. Procédé pour surveiller la formation de biofilms sur les surfaces dans des systèmes aquatiques, qui comprend l'incorporation dans le système aquatique d'un appareil d'échantillonnage comprenant un conduit qui forme un canal pour un écoulement d'eau et qui possède au moins un orifice (2) dans sa paroi, un plot (17) présentant une surface de test (19) bio-salissable, et des moyens de retenue (13) afin de retenir le plot dans l'orifice en relation fixe par rapport au conduit, de sorte que la surface de test est sensiblement en affleurement avec la paroi du conduit et qu'elle est exposée à l'eau dans le canal, et
le procédé comprenant une opération consistant à retenir le plot dans l'appareillage d'échantillonnage pendant un intervalle de temps pour le test, caractérisé en ce que l'on enlève ensuite le plot hors de l'orifice en utilisant un élément d'enlèvement (21) pour le plot, le plot et l'élément d'enlèvement ayant des organes de montage appropriés pour monter rigidement le plot sur l'élément d'enlèvement en vue de l'enlèvement hors de l'appareil, et
en ce qu'on collecte et qu'on analyse les bactéries provenant de la surface de test du plot.
